# EUROPEAN PATENT SPECIFICATION

(11) **EP 2 608 764 B1**
(45) Date of publication and mention of the grant of the patent: **30.12.2015**
(21) Application number: 11745987.5
(22) Date of filing: 22.08.2011
(51) Int. Cl.: A61K 8/29, A61K 8/40, A61Q 17/04, A61Q 19/00, A61Q 19/02, A61K 8/27, A61K 8/02

(54) **A PERSONAL CARE COMPOSITION COMPRISING AN INORGANIC PIGMENT AND AN ORGANIC DYE**
KÖRPERPFLEGEZUSAMMENSETZUNG MIT EINEM ANORGANISCHEN PIGMENT UND EINEM ORGANISCHEN FARBSTOFF
COMPOSITION DE SOINS PERSONNELS COMPRENANT UN PIGMENT INORGANIQUE ET UN COLORANT ORGANIQUE

(30) Priority: 24.11.2010 EP 10192309; 24.08.2010 IN MU23612010
(43) Date of publication of application: 03.07.2013
(73) Proprietor: Unilever N.V., 3013 AL Rotterdam (NL); Unilever PLC, London, Greater London EC4Y 0DY (GB)
(72) Inventor: RAJAGOPAL, Ramasubramaniam, Whitefield Bangalore 560 066 (IN); ROY, Arindam, Whitefield Bangalore 560 066 (IN)
(74) Representative: Corsten, Michael Allan
(86) International application number: PCT/EP2011/064352
(87) International publication number: WO 2012/025477

(56) References cited:
- EP-A2- 0 266 124
- EP-A2- 1 371 359
- US-A1- 2005 019 285

## Description

### Technical Field

The invention relates to personal care compositions for improved skin appearance especially for dark skinned consumers.

### Background of the invention

People all over the world desire to have healthy skin which not only appears young but also to have a pleasing appearance with even skin tone. One of the ways they achieve this is by ensuring minimal damage to the skin by protecting it against harsh environmental conditions like heat, humidity, sunlight, pollution and dust. In addition to protecting the skin, consumers also like to apply cosmetics to minimize the appearance of irregularities on the skin. In tropical countries where people generally have dark skin, there is a desire to have lighter skin appearance. People who live far from the tropical areas e.g. the Caucasian people who generally have lighter skin, prefer to have an even tanned tone of their skin. Any exposure of their skin to sunlight, often leads to blotchy skin, referred to as freckles and in some cases they experience hyperpigmentation in localized areas of the skin. In all of the above cases, people generally rely on cosmetic solutions to their skin protection and appearance problems.

In providing photoprotection of the skin, manufacturers, heretofore have concentrated mostly on using ingredients which block or absorb ultra-violet (UV) rays of the sun from reaching the skin. By improved skin appearance, consumers expect an even skin tone, which mask the irregularities of the skin. Further dark skinned consumers, desire that the base colour of the skin changes to a lighter skin tone.

In order to provide a solution to the above problem the present inventors have been working intensively on developing a composition that provides the desired skin appearance. They found that in order to deliver this, it is important to have a composition that contains ingredients that have two different properties i.e. (i) one material which ensures reflection of incident light predominantly in the red region and (ii) another material which ensures absorption of incident light predominantly in the blue region. This unique combination ensures delivery of not only an even-skin without it being unnaturally white or pale but ensures photoprotection. In order to deliver this, the inventors have invented a selective combination of an inorganic pigment particle that has a specific light scattering property and an organic dye that has a specific light absorption property. They found that this is especially effective when used by dark skinned consumers.

Compositions that combine inorganic particles and organic dyes are known. EP 1 371 359 (Biersdorf, 2003), discloses a cosmetic or dermatological composition comprising a titanium or iron oxide pigment with a mean particle diameter above 500 nm, an organic dye, and one or more UV filters selected from asymmetrically substituted triazines, phenylene-1,4-bis(2-benzimidazyl)-3,3',5,5'-tetrasulfonic acid and its salts and 2,2'-methylenebis(6-(2H-benzotriazol-2-yl)-4-(1,1,3,3-tetramethylbutyl)phenol).

US 2002/0176831 (Avon) discloses a coloured cosmetic composition comprising: an emulsion formed of an aqueous phase and an oil phase, said aqueous phase having a hydrophilic colourant which is preferably a dye or an inorganic particle; and a pearlescent colorant which is preferably selected from a group of inorganic colourant particulates being present in the oil phase or external to the emulsion.

US 6 080 415 (L'Oreal, 2000) discloses a cosmetic make-up product comprising two components packaged separately, the first component having a photochromic colouring agent which is selected from a list of inorganic particles or organic dyes which is capable of producing at least one colour in the presence of uv light and the second component comprising an agent that screens uv light.

While there is general mention of use of inorganic pigments and dyes in the above cited publications, none of the them disclose that a combination of selective inorganic pigment particle of specified size that has a specific light scattering property (reflection of incident light predominantly in the red region) and selected organic dyes that have a specific light absorption property (absorption of incident light predominantly in the blue region) provides for a highly pleasing even skin appearance.

It is thus an object of the present invention to provide for a personal care composition that gives the skin a pleasing lighter appearance while ensuring protection of the skin against light rays.

### Summary of the invention

According to the present invention there is provided a personal care composition for improved skin appearance comprising
(i) 0.5 to 10% of inorganic pigment particles in the size range of 400 to 800 nm which scatter greater than 50% of light in the wavelength range 550 to 700 nm with respect to total scattered light in the range of 400 to 700 nm, selected from titanium dioxide or zinc oxide and
(ii) 0.01 to 0.5% of organic dye which absorbs greater than 50% of light in the wavelength range 400 to 550 nm with respect to total absorbed light in the range of 400 to 700 nm selected from FDC Red 4, FDC Red 1 or DC Brown.

### Detailed description of the invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. The disclosure of the invention as found herein is to be considered to cover all embodiments as found in the claims as being multiply dependent upon each other irrespective of the fact that claims may be found without multiple dependency or redundancy.

"Personal Care Composition" as used herein, is meant to include a composition for topical application to skin of mammals, especially humans. Such a composition is preferably a leave-on composition. It includes any product applied to a human body for getting improved appearance, cleansing, odor control, photoprotection or general aesthetics and is especially useful for providing improved appearance of human skin. The composition of the present invention can be in the form of a liquid, lotion, cream, foam, scrub, gel, soap or toner, or applied with an implement or via a face mask, pad, patch or spray. Non-limiting examples of personal care compositions include leave-on compositions like skin lotions, creams, antiperspirants, deodorants, depilatories, lipsticks, foundations, mascara, sunless tanners and sunscreen lotions. "Skin" as used herein is meant to include skin on the face and body (e.g., neck, chest, back, arms, underarms, hands, legs, buttocks and scalp). The composition is preferably not a wash-off product since in these cases, the particle and dye are not deposited in sufficient amounts on the skin to enable the desired improvement in skin appearance.

The present invention relates to a personal care composition for improved skin appearance, especially among dark skinned consumers. The composition comprises selective inorganic pigment particles which scatter greater than 50% of light in the wavelength range 550 to 700 nm with respect to total scattered light in the range of 400 to 700 nm. This is to mean that the inorganic pigment particles scatter light more in the red region of the visible light spectrum (550 to 700 nm) as compared to other regions of the visible light spectrum (400 to 550 nm). Thus, when visible light is incident on the inorganic pigment particles used in the composition of the invention, the light is predominantly scattered in the red region (550 to 700 nm) of the spectrum. The composition comprises 0.5 to 10%, preferably 1 to 5% by weight of the inorganic pigment particles which are selected form zinc oxide or titanium dioxide. The particle size of the inorganic pigment particles are in the range of 400 to 800 nm. By particle size is meant particle diameter.

The composition of the invention comprises an organic dye which absorbs greater than 50% of light in the wavelength range 400 to 550 nm with respect to total absorbed light in the range of 400 to 700 nm selected from FDC Red 4, FDC Red 1 or DC Brown (e.g. Chocolate Brown TAS). Combinations of these dyes, sometimes available commercially as mixtures, are also within the scope of the invention. This is to mean that the organic dye absorbs light more in the blue region of the visible light spectrum (400 to 550 nm) as compared to other regions of the visible light spectrum (550 to 700 nm). Thus, when visible light is incident on the organic dye used in the composition of the invention, the light is predominantly absorbed in the blue region (400 to 550 nm) of the spectrum. The composition comprises 0.01 to 0.5%, preferably 0.05 to 0.3% by weight of the organic dye.

It is especially useful to use by consumers who are more dark skinned. By dark skinned is meant those whose skin has a L* value of 30 to 65, where L* is the lightness of skin colour in the CIE L*a*b* scale.

A suitable way of incorporating the selective particle and selective dye in the composition of the invention is to mix the two ingredients at suitable steps in process of preparation of the composition. Alternately, it is also possible to prepare the particles in such a way that they are coated with the selected dye. The particles thus coated with the dye could be incorporated in the composition of the invention.

The composition of the present invention is preferably delivered to the external surface of the human body where there are visible irregularities on the skin through a cosmetically acceptable base. Suitable cosmetically acceptable bases are cream, lotion, gel or emulsion.

Cosmetic compositions may be prepared using different cosmetically acceptable emulsifying or non-emulsifying systems and vehicles. A highly suitable base is a cream. Vanishing creams are especially preferred. Vanishing cream bases generally comprise 5 to 25% by weight fatty acid and 0.1 to 10% by weight soap. Vanishing cream base gives a highly appreciated matty feel to the skin. C₁₂ to C₂₀ fatty acids are especially preferred in vanishing cream bases, further more preferred being C₁₄ to C₁₈ fatty acids. The most preferred fatty acid is stearic acid. The fatty acid in the composition is more preferably present in an amount in the range of 5 to 20% by weight of the composition. Soaps in the vanishing cream base include alkali metal salt of fatty acids, like sodium or potassium salts, most preferred being potassium stearate. The soap in the vanishing cream base is generally present in an amount in the range of 0.1 to 10%, more preferably 0.1 to 3% by weight of the composition. Generally the vanishing cream base in cosmetic compositions is prepared by taking a desired amount of total fatty matter and mixing with potassium hydroxide in desired amounts. The soap is usually formed in-situ during the mixing. The composition of the invention preferably comprises water. Water is preferably present in 35 to 90%, more preferably 50 to 85% by weight of the composition.

The composition of the invention may additionally comprise a skin lightening agent. The skin lightening agent is preferably chosen from one or more of one or more of a vitamin B3 compound or its derivative e.g. niacin, nicotinic acid, niacinamide. Any other well known skin lightening agents e.g. aloe extract, ammonium lactate, azelaic acid, kojic acid, butyl hydroxy anisole, butyl hydroxy toluene, citrate esters, 2,5-dihydroxybenzoic acid and its derivatives, ellagic acid, fennel extract, green tea extract, hydroquinone, 4-hydroxyanisole and its derivatives, 4-hydroxy benzoic acid derivatives, hydroxycaprylic acid, lemon extract, linoleic acid, magnesium ascorbyl phosphate, salicylic acid, vitamins like vitamin B6, vitamin B12, vitamin C, vitamin A, a dicarboxylic acid, resorcinol derivatives, hydroxycarboxylic acid like lactic acid and their salts e.g. sodium lactate, and mixtures thereof, may also be incorporated in the composition of the invention. Vitamin B3 compound or its derivative e.g. niacin, nicotinic acid, niacinamide are the more preferred skin lightening agent as per the invention, most preferred being niacinamide. Niacinamide, when used, is preferably present in an amount in the range of 0.1 to 10%, more preferably 0.2 to 5% by weight of the composition. Without wishing to be bound by theory, it is believed that the composition of the invention comprising the selective particle and dye when combined with a suitable skin lightening agent provides for synergistic improvement in skin appearance.

The cosmetic composition may preferably additionally comprise one or more UV sunscreens. The UV sunscreens may be inorganic or organic.

A wide variety of organic sunscreen agents are suitable for use in combination with the essential ingredients of this invention. Suitable UV-A / UV-B sunscreen agents include 2-hydroxy-4-methoxybenzophenone, octyldimethyl-p-aminobenzoic acid, digalloyltrioleate, 2,2-dihydroxy-4-methoxybenzophenone, ethyl-4-(bis(hydroxypropyl)) aminobenzoate, 2-ethylhexyl-2-cyano-3,3-diphenylacrylate, 2-ethylhexylsalicylate, glyceryl-p-aminobenzoate, 3,3,5-trimethylcyclohexylsalicylate, methylanthranilate, p-dimethylaminobenzoic acid or aminobenzoate, 2-ethylhexyl-p-dimethyl- amino-benzoate, 2-phenylbenzimidazole-5-sulfonic acid, 2-(p-dimethylaminophenyl)-5-sulfonicbenzoxazoic acid, 2-ethylhexyl-p-methoxycinnamate, butylmethoxydibenzoylmethane, 2-hydroxy-4-methoxybenzophenone, octyldimethyl-p-aminobenzoic acid and mixtures thereof. Most suitable organic sunscreen are 2-ethylhexyl-p-methoxycinnamate and butylmethoxydibenzoylmethane.

A safe and effective amount of sunscreen may be used in the compositions useful in the subject invention. The composition preferably comprises from about 0.1 % to about 10%, more preferably from about 0.1 % to about 5% by weight of a sunscreen agent.

The composition according to the invention may also comprise other diluents. The diluents act as a dispersant or carrier for other materials present in the composition, so as to facilitate their distribution when the composition is applied to the skin.

Diluents other than water can include liquid or solid emollients, solvents, humectants, thickeners and powders.

The cosmetically acceptable base is usually from 10 to 99.9%, preferably from 50 to 99% by weight of the composition, and can, in the absence of other cosmetic adjuncts, form the balance of the composition.

The compositions of the present invention can comprise a wide range of other optional components. The CTFA Cosmetic Ingredient Handbook, Second Edition, 1992, which is incorporated by reference herein in its entirety, describes a wide variety of non-limiting cosmetic and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents and skin healing agents.

The composition is formulated in any known format, more preferred formats being creams or lotions.

The composition of the invention may comprise a conventional deodorant base as the cosmetically acceptable carrier. By a deodorant is meant a product in the stick, roll-on, or propellant medium which is used for personal deodorant benefit e.g. application in the under-arm area which may or may not contain anti-perspirant actives.

Deodorant compositions can generally be in the form of firm solids, soft solids, gels, creams, and liquids and are dispensed using applicators appropriate to the physical characteristics of the composition.

Deodorant compositions which are delivered through roll-ons generally comprise a liquid carrier. Such liquid carrier can be hydrophobic or comprise a mixture of both hydrophilic and hydrophobic liquids. They may be in the form of an emulsion or a microemulsion. The liquid carrier or mixture of carriers often constitutes from 30 to 95% by weight of the composition and in many instances from 40 to 80%.

Hydrophobic liquid carriers commonly can comprise one or more materials selected within the chemical classes of siloxanes, hydrocarbons, branched aliphatic alcohols, esters and ethers that have a melting point not higher than 25°C and a boiling point of at least 100°C.

Hydrophilic carrier liquids that can be employed in compositions herein commonly comprise water and/or a mono or polyhydric alcohol or water-miscible homologue. Monohydric alcohols often are short chain, by which is meant that they contain up to 6 carbons, and in practice are most often ethanol or sometimes iso-propanol. Polyhydric alcohols commonly comprise ethylene or propylene glycol, or a homologue can be employed such as diethylene glycol.

The compositions that remain in liquid form can be applied employing conventional applicators such as a roll-on or by being pumped or squeezed through a spray-generating orifice. Such compositions may be thickened, for example using one or more thickeners described subsequently herein.

Compositions that are firm solids, commonly obtained by use of a gellant or structurant, can be applied employing a stick applicator and soft solids, gels and creams can be applied employing an applicator having a dispensing head provided with at least one aperture through which the soft solid, gel or cream can be extruded under mild pressure.

Suitable thickeners or gellants that may be used for achieving this is by use of water-soluble or dispersible materials of higher viscosity, including various of the emulsifiers, and/or thickened or gelled with water-soluble or water-dispersible polymers including polyacrylates, and water-soluble or dispersible natural polymers, such as water-soluble polysaccharide or starch derivatives, such as alginates, carageenan, agarose and water-dispersible polymers include cellulose derivatives.

The concentration of such polymers in the water-immiscible liquid is often selected in the range of from 1 to 20% by weight, depending on the extent of thickening or structuring required, and the effectiveness of the chosen polymer in the liquid/mixture.

One class of structurant which is desirable by virtue of its long standing proven capability to produce firm solids and more recently in making soft solids, comprises waxes. Herein, the term wax is employed to encompass not only materials of natural origin that are solid with a waxy feel and water-insoluble at 30-40°C, but melt at a somewhat higher temperature, typically between 50 and 95°C, such as beeswax, candelilla or carnauba wax, but also materials having similar properties. Such other waxes include hydrocarbon waxes, eg paraffin wax, mineral wax and microcrystalline wax; synthetic waxes, such as polyethylene of 2000 to 10000 daltons; waxy derivatives or waxy components of natural waxes

Mixtures of materials within each class of gellant/ structurant can be employed.

When a deodorant composition employed herein comprises an aerosol composition, it contains a propellant in addition to a base composition as described herein above, commonly in a weight ratio of from 95:5 to 40:60, and in many formulations, the weight ratio is from 90:10 to 50:50.

The propellant is conveniently a low boiling point material, typically boiling below -5°C, for example an alkane such as propane, butane or isobutane, and possibly containing a fraction of pentane or isopentane, or a hydrofluorocarbon or fluorocarbon of similar carbon content. During filling of the aerosol canister, the propellant gas is liquified by virtue of the elevated pressure that is generated therein.

According to yet another aspect of the present invention there is provided use of a composition as claimed in any one of the preceding claims for improved appearance of dark skin. The use is preferably non-therapeutic use.

The invention is now further described by way of the following non-limiting examples.

### EXAMPLES

### Example 1 to 3: Reflectance (measure of skin appearance) provided by composition as per the invention (example 1) as compared to examples outside the invention (examples 2 and 3)

The following compositions (as shown in table 1) were prepared.

**Table 1**

| Examples | 1 Wt% | 2 Wt% | 3 Wt% |
|---|---|---|---|
| Hysteric acid | 4.0 | 4.0 | 4.0 |
| Glycerine | 1.0 | 1.0 | 1.0 |
| Isopropyl myristate | 3.0 | 3.0 | 3.0 |
| Sodium hydroxide | 0.05 | 0.05 | 0.05 |
| Cetyl alcohol | 0.2 | 0.2 | 0.2 |
| Silicone oil | 0.5 | 0.5 | 0.5 |
| Methyl paraben + propyl paraben | 0.3 | 0.3 | 0.3 |
| Phenoxy ethanol | 0.6 | 0.6 | 0.6 |
| Disodium EDTA | 0.04 | 0.04 | 0.04 |
| Parsol 1789 | 0.4 | 0.4 | 0.4 |
| Parsol MCX | 1.25 | 1.25 | 1.25 |
| Particle* | 3.0 | 3.0 | 0.0 |
| Dye** | 0.26 | 0.0 | 0.26 |
| Water | To 100 | To 100 | To 100 |

| | | | |
|---|---|---|---|
| * Particle used was titanium oxide of particle size in the 0.4 to 0.8 micron range. This particle scatters about 64 % in the wavelength range of 550 to 700 nm with respect to total scatter in the 400 to 700 nm range. **Dye used was Chocolate Brown TAS which absorbs about 90% in the wavelength range of 400 to 550 nm with respect to total absorption in the 400 to 700 nm range. | | | |

The reflectance of the various examples 1 to 3 were measured using the following procedure:
Reflectance measurements: Measured amounts (2 mg/cm²) of the composition were applied on skin like substrate. The substrate was a 3M transpore tape glued to a brown paper (L* of 57.7, a* of 5.2 and b* of 9.5) whose colour was similar to human skin (L* 56.4, a* of 9.7 and b* of 20.4) for the measurements. Diffused reflectance was measured using a spectrophotometer (model Macbeth-7000Eye), equipped with integrating sphere. The data on the reflectance provided by the composition of examples 1 to 3 are summarized in table 2 along with the reflectance of a typical dark skinned consumer without any composition applied (Background).

The reflectance of a fair skinned individual compared to that of a dark skinned individual is generally higher in the red region of the reflectance spectra as compared to that of the blue region. Hence, it is generally desired, by dark skinned consumers, for good skin appearance that the difference in the reflectance in the blue region provided by the composition with respect to the background is low while the reflectance provided by the composition in the red region is high with respect to the background, thereby to lighten the skin.

**Table 2**

| % Reflectance | Background | Example 1 | Example 2 | Example 3 |
|---|---|---|---|---|
| 410 nm | 18.2 | 16.4 | 11.8 | 24.2 |
| 450 nm | 19.0 | 16.8 | 12.0 | 24.0 |
| 500 nm | 21.7 | 21.8 | 15.9 | 25.7 |
| 550 nm | 23.7 | 27.0 | 19.6 | 27.0 |
| 600 nm | 27.0 | 33.9 | 25.2 | 30.0 |
| 650 nm | 36.4 | 39.6 | 33.3 | 39.7 |
| 700 nm | 48.8 | 55.7 | 49.6 | 50.7 |

The data in table 2 indicates that only the composition as per the invention (example 1) provides the desired skin appearance while the other two compositions do not.

Examples 4 to 6: Use of particles with sizes within and outside the invention Compositions similar to example 1 were prepared except that the particle size in each of the compositions were different.

Example 4 was similar to example 1 in that the particle used was titanium oxide of particle size in the 0.4 to 0.8 micron range. Example 5 used titanium dioxide of particle size in the 0.01 to 0.25 micron range. Example 6 used titanium dioxide of particle size 1 to 5 microns. The reflectance of the compositions of example 4 to 6 were measured using the procedure described for examples 1 to 3 and the data is summarized in table 3.

**Table 3**

| % Reflectance | Background | Example 4 | Example 5 | Example 6 |
|---|---|---|---|---|
| 410 nm | 15.8 | 16.2 | 12.0 | 13.6 |
| 450 nm | 16.2 | 16.6 | 12.6 | 14.0 |
| 500 nm | 17.9 | 21.6 | 15.9 | 18.3 |
| 550 nm | 19.0 | 25.9 | 18.7 | 22.3 |
| 600 nm | 21.8 | 31.1 | 23.1 | 27.8 |
| 650 nm | 31.2 | 36.9 | 31.1 | 34.5 |
| 700 nm | 46.2 | 53.0 | 47.6 | 51.3 |

The data in table 3 indicates that the reflectance provided by the composition of the invention (example 4) is as desired by a dark skinned person, i.e. higher in the red region of the reflectance spectra as compared to that of the blue region, with respect to the background. Such benefit is not observed when either particle sizes smaller or larger than the claimed range are used (examples 5 and 6).

## Claims

1. A personal care composition for improved skin appearance comprising
(i) 0.5 to 10% of inorganic pigment particles in the size range of 400 to 800 nm which scatter greater than 50% of light in the wavelength range 550 to 700 nm with respect to total scattered light in the range of 400 to 700 nm selected from titanium dioxide or zinc oxide, and
(ii) 0.01 to 0.5% of organic dye which absorbs greater than 50% of light in the wavelength range 400 to 550 nm with respect to total absorbed light in the range of 400 to 700 nm selected from FDC Red 4, FDC Red 1 or DC Brown.

2. A composition as claimed in claim 1 wherein said composition is a leave-on composition.

3. A composition as claimed in claim 1 or 2 comprising 5 to 25% by weight fatty acid and 0.1 to 10% by weight soap.

4. Use of a composition as claimed in any one of the preceding claims for improved appearance of dark skin having a L* value of 30 to 65, where L* is the lightness of skin colour in the CIE L*a*b* scale, said improved appearance evidenced by a low difference in the reflectance provided by the composition with respect to the background in the blue region while providing a high difference in the reflectance by the composition with respect to the background in the red region.

5. A composition according to any one of claims 1 to 3 for use in a method for lightening the skin of a consumer who is dark skinned, having a L* value of 30 to 65, where L* is the lightness of skin colour in the CIE L*a*b* scale, the use comprising applying to the skin the composition of any one of claims 1 to 3.

6. A composition according to any one of claims 1 to 3 for use in a method for lightening skin.

## Patentansprüche

1. Körperpflegezusammensetzung für verbessertes Aussehen der Haut, umfassend
(i) 0,5 bis 10% anorganische Pigmentteilchen im Größenbereich zwischen 400 bis 800 nm, welche mehr als 50% Licht in dem Wellenlängenbereich von 550 bis 700 nm streuen, bezogen auf das gesamte gestreute Licht in dem Bereich von 400 bis 700 nm, ausgewählt aus Titandioxid oder Zinkoxid, und
(ii) 0,01 bis 0,5% organischen Farbstoff, welcher mehr als 50% Licht in dem Wellenlängenbereich von 400 bis 550 nm absorbiert, bezogen auf das gesamte absorbierte Licht in dem Bereich von 400 bis 700 nm, ausgewählt aus FDC Rot 4, FDC Rot 1 oder DC Braun.

2. Zusammensetzung gemäß Anspruch 1, wobei die Zusammensetzung eine zum Verbleiben auf der Haut ist.

3. Zusammensetzung gemäß Anspruch 1 oder 2, umfassend 5 bis 25 Gew.-% Fettsäure und 0,1 bis 10 Gew.-% Seife.

4. Verwendung einer Zusammensetzung gemäß einem der vorhergehenden Ansprüche für besseres Aussehen von dunkler Haut mit einem L*-Wert von 30 bis 64, wobei L* die Helligkeit der Hautfarbe in der CIE L*a*b* Skala ist, wobei das verbesserte Aussehen durch einen geringen Unterschied im Reflexionsgrad nachgewiesen wird, der von der Zusammensetzung im blauen Gebiet in Bezug auf den Hintergrund hervorgerufen wird, während von der Zusammensetzung im roten Gebiet in Bezug auf den Hintergrund ein großer Unterschied hervorgerufen wird.

5. Zusammensetzung gemäß irgendeinem der Ansprüche 1 bis 3 zur Verwendung in einem Verfahren zum Aufhellen der Haut eines dunkelhäutigen Verbrauchers mit einem L*-Wert von 30 bis 64, wobei L* die Helligkeit der Hautfarbe in der CIE L*a*b* Skala ist, wobei die Verwendung das Aufbringen der Zusammensetzung gemäß irgendeinem der Ansprüche 1 bis 3 auf die Haut umfasst.

6. Zusammensetzung gemäß irgendeinem der Ansprüche 1 bis 3 zur Verwendung in einem Verfahren zum Aufhellen von Haut.

## Revendications

1. Composition pour l'hygiène personnelle pour une apparence de peau améliorée comprenant
(i) de 0,5 à 10 % de particules de pigment inorganique dans l'intervalle de taille de 400 à 800 nm qui diffusent plus de 50 % de lumière dans l'intervalle de longueur d'onde de 550 à 700 nm par rapport à la lumière totale diffusée dans l'intervalle de 400 à 700 nm choisies parmi le dioxyde de titane ou l'oxyde de zinc, et
(ii) de 0,01 à 0,5 % de colorant organique qui absorbe plus de 50 % de lumière dans l'intervalle de longueur d'onde de 400 à 550 nm par rapport à la lumière totale absorbée dans l'intervalle de 400 à 700 nm choisi parmi le rouge FDC 4, le rouge FDC 1 ou le brun DC.

2. Composition selon la revendication 1, dans laquelle ladite composition est une composition à laisser poser.

3. Composition selon la revendication 1 ou 2 comprenant de 5 à 25 % en poids d'acide gras et de 0,1 à 10 % en poids de savon.

4. Utilisation d'une composition selon l'une quelconque des revendications précédentes pour une apparence améliorée de peau foncée présentant une valeur L* de 30 à 65, où L* est la clarté de la couleur de peau dans l'échelle CIE L*a*b*, ladite apparence améliorée étant mise en évidence par une faible différence du coefficient de réflexion fourni par la composition par rapport à l'arrière-plan dans la région bleue tout en fournissant une différence élevée du coefficient de réflexion par la composition par rapport à l'arrière-plan dans la région rouge.

5. Composition selon l'une quelconque des revendications 1 à 3 pour une utilisation dans un procédé pour éclaircir la peau d'un consommateur qui est de peau foncée, présentant une valeur L* de 30 à 65, où L* est la luminosité de la couleur de peau dans l'échelle CIE L*a*b*, l'utilisation comprenant l'application sur la peau de la composition selon l'une quelconque des revendications 1 à 3.

6. Composition selon l'une quelconque des revendications 1 à 3 pour une utilisation dans un procédé pour éclaircir la peau.
